# EUROPEAN PATENT APPLICATION

(11) **EP 3 763 821 A1**
(43) Date of publication of application: **13.01.2021**
(21) Application number: 20184356.2
(22) Date of filing: 07.07.2020
(51) Int. Cl.: C12Q 1/04, G01N 21/88, G01J 3/44, G01N 21/64, G01N 21/63

(54) **MOBILE DEVICE FOR NON-DESTRUCTIVE FLUORESCENCE RESOLUTION, DISPLAY AND QUANTIFICATION OF MICROORGANISMS ON THE SURFACE OF MATERIALS**

(30) Priority: 09.07.2019 CZ 20190459
(71) Applicant: Botanicky ustav Akademie ved CR, v.v.i., 25243 Pruhonice (CZ)
(72) Inventor: Marsalek, Blahoslav, 64300 Brno, Chrlice (CZ); Klecka, Jan, 76005 Zlin (CZ); Zezulka, Stepan, 66601 Nelepec-Zernuvka (CZ); Marsalkova, Eliska, 64300 Brno, Chrlice (CZ)
(74) Representative: Dadej, Leopold

(57) **Abstract**

The invention is a mobile device for non-destructive fluorescence differentiation, imaging, and quantification of microorganisms, in particular moulds, bacteria, algae, cyanobacteria, mosses, and lichens, on a material surface comprising an excitation radiation source for excitation of the detected material surface and a camera for fluorescence imaging of the excited material surface provided with a memory module for storing images from the camera,
characterized in that it contains an excitation optical chamber with an aperture for placement on the detected surface of the material in which a set of excitation radiation sources, a camera, a set of optical emission filters movable individually into the camera's field of view and transmitting wavelengths corresponding to the fluorescence signals elicited by the excitation radiation of the excitation radiation sources and characteristic of individual groups of organisms, and an emission filter controller for moving the optical emission filters into the camera's field of view are enclosed.

## Description

### Technical field

The invention relates to a device for non-destructive detection and quantification of organisms that decompose the surfaces of materials and are able to penetrate to the depth of these materials, e.g. concrete, facades, wood, plastics, but also natural stone in case of sculptures, etc., thereby causing their destruction.

### Background of the invention

Microbial contamination of various surfaces presents a real hygienic, aesthetic, and technological problem, and therefore, new, more accurate, and the simplest possible methods for detecting the presence, differentiation, and quantification of organisms growing on surfaces have been constantly developed. Photoautotrophic or mixotrophic communities of cyanobacteria, algae, bacteria, and fungi occur naturally on various surfaces in nature, from the soil surface through, for example, the surface of a tree bark to the surface of stones and rocks, where a necessary condition is at least temporary availability of water and atmospheric pollution as a source of nutrients that these organisms can use very efficiently. However, through their metabolic activity, they can also destroy, corrode the original substrates and materials on which they grow (Zanardini et al., 2019). Cyanobacteria (Gaylarde et al., 2018) or fungi (Boniek et al., 2017; Mejia et al., 2019) and lichens (Sohrabi et al., 2017) are often responsible for biocorrosion. In the anthroposphere, suitable surfaces for these communities are surfaces and structures of buildings, whether made of wood, natural rocks, or artificial building materials, such as concrete. Therefore, in addition to climatic conditions, microorganism communities can significantly contribute to the degradation - deterioration of surface quality and structural integrity of the building material, cause its (bio)corrosion (Warscheid and Braams, 2000), and increase the risk of its disintegration and degradation of the whole building, or increase the costs of its repairs and maintenance. Microbial communities create problems, such as discoloration of surfaces, water retention, or subsequent mechanical frost destruction, surface and deep corrosion of materials, increasing their fragility, etching, blistering of plasters, physical damage to surfaces, and increasing permeability for water and thus for other organisms, etc.

To correctly estimate the damage and method of treatment of materials, it is necessary to know as accurately as possible the extent and composition of the microbial community contaminating the surface layers. There are a number of methods for detecting, distinguishing, and quantifying biofilm-forming microorganisms. An overview of the basic groups of methods is described, for example, in Sanmartin et al. (2018), from which it is clear that the essence of the current methods is taking samples by scraping and transferring them to the laboratory to determine the composition.

Among the most accurate in determining the taxonomic identity of microorganisms are molecular methods aimed at detecting specific biomolecules - nucleic acids, such as DNA and RNA segments carrying specific genes (Mejia et al., 2019; Zanardini et al., 2019), enzymes, or secondary metabolites (Boniek et al., 2017; Sohrabi et al., 2017). However, these methods are currently still very expensive and demanding on the laboratory equipment. In addition, they require not only the physical collection of the material but often also the subsequent, time-consuming, professionally and materially demanding cultivation of the collected microorganisms in laboratory conditions.

Another group of detection methods consists of previously commonly used microscopic observations using optical or electron microscopy (Gaylarde et al., 2017; Gaylarde et al., 2018), or newer methods using the optical properties of the examined microorganisms - absorbance, reflectance, and spectroscopic properties, such as luminescence or fluorescence. While microscopy usually also requires destructive sampling, other optical methods can now be applied in situ, they do not necessarily need to be destructive, and their complexity and accuracy depend on the particular methodology used and the parameter being evaluated. Other techniques usable for direct observation and study of the influence of microorganisms on building materials or materials of cultural heritage sites include direct observation, optical and fluorescence microscopy, scanning microscopy (Nuhoglu et al 2017), confocal laser scanning microscopy, atomic absorption spectroscopy, differential thermal analysis, electron paramagnetic resonance (EPR), and many others, mentioned by e.g. Gaylarde et al. (2017, 2018). However, the above-mentioned methods require sampling, for example by scraping, which may lead to the destruction of the building's surface. In many cases, especially in case of listed buildings, however, mechanical damage by material collection is undesirable, and therefore, the goal is to use non-destructive and non-contact methods for the detection of biotic contamination.

In case of fluorescent methods, it is possible to use autofluorescence inherent in a given microorganism or to observe the fluorescence of artificial dyes and chemical probes caused by contact of such a substance with cells of microorganisms or, as in case of molecular methods, with specific biomolecules (Gonzalez-Perez et al., 2017). Properties of the fluorescence signal, such as intensity or emission spectrum and shifts in it, can be used not only to detect the presence of microorganisms but also to distinguish them into taxonomic groups (at least bacteria, cyanobacteria, algae, and moulds), determine viability, or quantify them. The presence of organisms (autofluorescence of algae, cyanobacteria), induced fluorescence of metabolites (ATP, DNA polysaccharides, etc.), or metabolic activity of microorganisms (photosynthesis, hydrolases, oxidases, etc.) can be detected fluorescently. However, these measurements also require expensive laboratory instruments and stable analytical conditions.

Therefore, we focused on in-situ methods that can be used to control biocorrosion and can serve to protect and prevent damage to buildings, i.e. early warning methods at the beginning of the development of microorganisms. The aim is to achieve a comprehensive view of groups of microorganisms (from moulds, fungi, and bacteria to algae and cyanobacteria) and their role in the biocorrosion process because biodeterioration is never caused by just one species of microorganism, instead, it is the result of the activity of a mixed microbial community at a certain stage of development on a given building.

There are very few in-situ methods that are inexpensive and yet detect a variety of microorganisms. The Fluorescence In-Situ Hybridization (FISH) method was tested (Gonzalez et al. 2014), which appears to be a cheap, relatively fast, and simple analytical method for the detection of fungi on marble. It has not been tested for other organisms yet.

Due to the need to detect microorganisms on surfaces, apparatuses dealing with this issue have been patented, for example, patent document number DE10244819 describes a device for the detection of fluorescent material on a technical surface that has the ability to detect fluorescent material on the surface, uses a non-destructive method of detection and differentiation of electrical contacts and impurities on them, such as plastic insulation residues. However, the apparatus is not able to detect or quantify individual groups of microorganisms, and the actual measurement is not possible under full lighting or sunlight in natural conditions, which reduces the sensitivity to the imaged fluorescence.

Another interesting apparatus that has been patented is a device for the detection of biotic contamination on a surface described in patent document DE19906047. It allows fluorescence in-situ detection of microbial cell residues, skin cell residues, residues of food, hair, sweat, sebum, and organic particles in general without further differentiation, on the principle of excitation of NAD(P)H or riboflavin. However, this apparatus is not able to either distinguish individual groups of organisms or to determine their ratio or quantify them. Another technical solution for the detection and quantification of growth communities is described in patent application US 2006/0275847A1, which describes an automated method for the detection of a biofilm with fluorescent particles on a solid surface using a confocal imaging system. The method includes scanning the biofilm using radiation, detecting fluorescence emitting particles in the biofilm, obtaining image data, and processing the data using a PC. The confocal imaging system includes a radiation source, focusing optics, a detector, and a multi-plane biofilm scanning device. The fluorescent particle may be a fluorescent protein (e.g. GFP) or a product of an enzymatic reaction (e.g. β-galactosidase, nitroreductase, alkaline phosphatase, β-lactamase) or a fluorescent compound. Preferably, the solid surface is a microtiter plate. A disadvantage of this solution is that it does not distinguish between individual groups of organisms, in principle it cannot quantify individual groups of organisms separately, instead, it monitors the biofilm and its growth as a whole.

An interesting technical solution is addressed in patent document US 2013/0266977 A1, which describes a method for detecting microorganisms on a solid surface based on the reaction of a substrate of a selected microbial enzyme to which a detectable, e.g. fluorescent, label is conjugated, and the microbial enzyme itself, which is present only in the presence of microorganisms. After the enzymatic reaction, the detectable label is released and provides a luminescent signal in the IR, VIS, or UV spectrum against a control of a known label concentration. The detection of microbes can take place in situ, without first dissolving the samples in a medium. This document further describes a non-fluid system for the detection of microorganisms on a solid surface comprising a sampling module with at least one test surface and one control surface, a radiation detecting device (source and detector), a PC module, and an imaging module. The test site contains an immobilised microbial enzyme substrate (e.g. N-acetyl-β-D-glucosaminide, N-acetyl-β-D-glucosamine, N,N-diacetyl-β-Dchitobioside, β-DN,N,N-triacetylchitotriose), to which a detectable, e.g. a fluorescent, label (e.g. 4-Methylumbelliferone) is conjugated. This apparatus is able to detect moulds and bacteria on which it is dominantly focused, but in the signal that the detector measures, there are also other microorganisms that the apparatus does not recognize. Qualitatively and quantitatively, it is able to recognize bacteria and moulds, but it is not able to simultaneously recognize in one image/position all groups of organisms that are important for the biocorrosion of materials (cyanobacteria, algae, bacteria, moulds, mosses, lichens, mineral and mechanical impurities).

The solution described in patent US7190457B2, which uses fluorescence for in-situ non-contact quantification of microorganisms on surfaces, can distinguish mechanical and mineral impurities on surfaces from microorganisms. This patent describes a real-time biofilm monitoring system comprising at least one optic fiber probe and an optoelectronic interface with a data collection system. The probe is provided with at least one excitation and at least one emission filter, wherein the probe detects the fluorescence of an inherent biomarker of the microorganism (e.g. amino acid, ATP, NADH, chlorophyll, bioluminescence), and is optionally provided with an excitation reference channel to correct spectral interference with non-biological materials (e.g. CaCO3, MgSO4). The US patent further discloses a method for detecting microorganisms using fluorescence, wherein the optical fiber is divided into two branches, where the first branch excites an analyses and the second branch detects the emission. The method can optionally be applied to samples of glass, polycarbonate, metal, or paint from a process fluid environment, heat exchanger, factory (e.g. producing microelectronic components or food, paper mills, or woodworking facilities) and other fluid handling devices. A disadvantage of this device is that it is not designed as a mobile device for field measurements without an electrical source and also that it quantifies chlorophyll as a sum and does not distinguish prokaryotic cyanobacteria from eukaryotic algae, which is very important from the point of view of biocorrosion because cyanobacteria are significantly more aggressive and active in decomposition of materials and penetration into the depths of materials than algae. The main disadvantage of this system is also a too small area of the detector that uses optical fibers, which is an absolutely crucial parameter for practical use because natural biofilms are very heterogeneous, and if the detector has a diameter as small as herein, it is suitable for, for example, pipe biofilms or internal surfaces of cooling towers. However, the natural communities involved in the decomposition of materials are naturally very diverse, and with this probe, dozens of sites would have to be measured to reliably evaluate the surface diversity, which is a significant practical disadvantage.

### Summary of the Invention

The above-mentioned limitations and disadvantages, especially the inability to selectively detect different groups of organisms through autofluorescence or fluorescence after a reaction with a fluorescent dye between the camera and the detected surface directly in the field and eliminate external disturbing effects, are solved with a mobile device for non-destructive fluorescence differentiation, imaging, and quantification of microorganisms, in particular moulds, bacteria, algae, cyanobacteria, mosses, and lichens, on the surface of materials, comprising
- an excitation radiation source for exciting the detected surface of the material and
- a camera for fluorescent imaging of the excited surface of the material provided with a memory module for storing images from the camera,
based on the fact that it contains an excitation optical chamber with an aperture for placement on the detected surface of the material, in which a set of excitation radiation sources, a camera, a set of optical emission filters movable individually into the camera's field of view and transmitting wavelengths corresponding to the fluorescence signals elicited by the excitation radiation of the excitation radiation sources and characteristic of individual groups of organisms, and an emission filter controller for moving the optical emission filters into the camera's field of view, are enclosed.

The device of the invention enables area imaging, group detection, and quantification of organisms that, due to their occurrence, growth, and metabolism, impair the mechanical, functional, and aesthetic qualities of the materials. The device detects and quantifies organisms based on fluorescent signals characteristic of individual groups of organisms, such as algae, cyanobacteria, diatoms, moulds, bacteria, mosses, and lichens.

A significant advantage over the previously known devices is that the device allows non-destructive analysis, it is not necessary to take a sample, and the device quantifies all organisms from one place, within one series of measurements, thus significantly improving the interpretive potential of the obtained data.

Depending on the filters used and as needed after spraying, the presence of organisms can be detected by fluorescence - autofluorescence, for example, chlorophyll, phycocyanin, etc., or induced fluorescence, for example, extracellular polysaccharides of bacteria, chitin of moulds and fungi, etc., or metabolic activity of these microorganisms, for example, photosynthesis, hydrolases, oxidases, etc. The detection of ATP and DNA present in each cell known from the previous solutions does not allow the selective detection of the organisms present, instead, it monitors the biofilm and its growth as a whole.

Thanks to a set of movable optical emission filters, the device of the invention has a universal use for the recognition and quantification of algae, cyanobacteria, bacteria, moulds, mosses, and lichens on the surfaces of various materials and can be used specifically for:
- Detection of the efficiency of biocidal treatment in cooling towers, waterworks, public areas of cellars, plasters, and surfaces of corridors and buildings, or natural surfaces of caves in order to identify, quantify, and prove whether the biocidal treatment was efficient. At the same time, in such cases, the apparatus is used for continuous monitoring and timing of further biocide application.
- The documentation and quantification of intentionally grown microbial biofilms, intended for water purification and decomposition of drugs and pesticides, where it is necessary to control sufficient biomass and diversity of organisms for their efficiency.

- Detection of dominant groups of microorganisms and their quantification on facades, statues, artistic artefacts, where microorganisms, through their growth, cause undesired black, yellow, green, red, or other discoloration and thereby reduce their aesthetic value. In this case, the apparatus can also be used preventively in the early warning mode because the apparatus is much more sensitive than the human eye due to the fluorescent excitation and distinguishes consortia of microorganisms already at the beginning of their growth.
- The most common use of the apparatus is in the field of biocorrosion materials, where microorganisms, through their metabolic activity, disrupt the mechanical structure and change the chemical composition of concrete, baked bricks and tiles, sandstone, limestone, wood, or, for example, plaster and facades. In this case, it is important to know not only how many but especially what groups of microorganisms occur on the surfaces because, for example, cyanobacteria, moulds, and lichens produce enzymes that etch the structure of materials and actively grow into cracks that they themselves actively create. Green algae, diatoms, or mosses cause aesthetic problems, but they are not as aggressive in deep corrosion of materials. Therefore, it is necessary for the apparatus to be able to recognize and quantify these organisms and thus make it possible to distinguish their harmfulness. According to the present invention, the apparatus is also able to spatially image the distribution and heterogeneity of individual groups of organisms, which is very important from a practical point of view when it is necessary to have a basis for deciding on a suitable prevention method or removal technology, including checking the efficiency of these technologies.

During the measurement, the optical emission filters are individually moved into the camera's field of view, whereby fluorescence of different wavelengths corresponding to the transmittance of the filters is gradually imaged during a single measurement. The optical emission filters can be arranged in a set, e.g. in a straight line, but will preferably be arranged in a circle, where the emission filter controller will be a rotating cylinder. In this case, the optical emission filters rotate into the camera's field of view by means of a rotating cylinder. The sequence of measurements of individual wavelengths is automatically repeated, thanks to which the measurements can be repeated, e.g. after moving to adjacent surfaces of the examined material, without the need to adjust the optical emission filters before each measurement. The emission filter controller is driven by a servomotor, and the replacement of the emission filters is automated and coordinated by the connected control unit.

To detect a specific group of organisms, it is necessary to generate appropriate excitation radiation of the required wavelengths and to use a suitable optical emission filter for the imaging. Thus, depending on the required degree of selectivity of the measurement, the camera for fluorescent imaging of the excited surface of the material performs repeated imaging of the measured surface using various combinations of excitation radiation and optical emission filters. E.g. autofluorescence of photosynthetic pigments is usually used for the detection of algae with a combination of excitation radiation of a wavelength of 465 nm and emission filter transmitting wavelengths of 680 nm, in case of cyanobacteria, an excitation radiation of a wavelength of 595 nm and emission filter transmitting wavelengths of 650 nm is suitable, in case of diatoms, excitation radiation of 520 nm, emission filter transmitting 680 nm, mosses are distinguishable from macroscopic photography by image analysis and spectra identical to the spectrum for algae. Moulds are stained using the fluorogenic reagent calcofluor with an excitation wavelength of 365 nm and a characteristic emission of 420 nm, and bacteria are stained using the fluorogenic reagent dansylhydrazine with excitation at 365 nm and emission of 520 nm. Lichens are detected by a combination of macroscopic photography and fluorescence spectra for algae, cyanobacteria, and moulds. In practice, not every position in the set of emission filters needs to be filled with a filter, and it is possible to leave free positions for mounting or replacement, i.e. for possible future filling with the required filter type for any other specific fluorescence measurements depending on the desired purpose of the measurement, e.g. metabolic activity, biocide efficiency evaluation, or quantification of the biological activity of biofilms for the degradation of pollutants. The optical emission filters in the device of the invention are preferably replaceable and substitutable for another type of optical excitation and/or emission filter for fluorescence detection, e.g. for measuring metabolic activity, evaluating biocide efficiency, quantifying biological activity of biofilms for pollutant degradation.

In order to be able to take sufficiently sensitive measurements even under full lighting or sunlight in natural conditions, the excitation radiation source and the camera are enclosed in an excitation optical chamber with an aperture for placement on the detected surface of the material. The camera in the excitation optical chamber images the surface of the material through the excitation chamber's aperture. This arrangement of the excitation source and the camera ensures the isolation of the excitation chamber from the surrounding light sources, minimizes their disturbing effects, and minimizes the disturbing reflectance of the glossy examined surfaces. The device thus detects fluorescence caused by excitation of even a small number of organisms.

The excitation chamber is defined by a rigid cover without disturbing optical properties to prevent the penetration of ambient light into the excitation optical chamber, wherein this cover is provided with a collar adaptable in shape to an uneven surface for pressing against the surface of the material. A suitable material for the collar is a flexible dark material without a shape memory, for example softened polyurethane foam. With such a collar, it is possible to cover unevenness even in the range of 1 to 2 cm. To press the cover of the excitation optical chamber more strongly against the measured surface, at least two support points are formed on opposite sides of the device of the invention connected to the device frame, e.g. a handle and a pressure pad located on the opposite side next to the screen.

In the excitation optical chamber, sources of excitation radiation are located above the aperture of the excitation chamber, wherein each optical emission filter corresponds to at least one source of excitation radiation, as mentioned above. In a preferred embodiment, multiple sources of excitation radiation are arranged in the excitation optical chamber for each optical emission filter. Preferably, the excitation radiation sources are arranged evenly on a circle coaxial with the aperture of the excitation chamber, e.g. in case of three excitation radiation sources at an angle of 120°, and inclined at an angle of 45° to 60° to the surface, in practice, an angle of inclination to the surface of 53° has proved to be advantageous. This minimizes unwanted reflectances, disturbing reflections from wet surfaces and other sources of optical artifacts, while ideally illuminating the uneven surface, thereby minimizing shading by the measured surface's unevenness that occurs when a single excitation radiation source is used or in the event of inappropriate placement of multiple such resources.

The excitation radiation sources for exciting the detected surface of the material may, in the basic embodiment, be LEDs emitting radiation of the desired wavelengths, which do not require additional optical correction. In case selective detection of microorganisms is required, it is necessary to place in the excitation chamber precise sources of excitation radiation that no longer need optical corrections by the filter, in a number corresponding to multiples of the number of optical emission filters used. For example, for 6 optical emission filters, in case of using three excitation radiation sources arranged in a circle at a distance of 120° for each filter, a total of 18 LEDs is needed.

In a preferred alternative embodiment, a set of universal broad-spectrum excitation radiation sources, e.g. broad-spectrum LEDs, is arranged in the excitation optical chamber, wherein each universal broad-spectrum excitation radiation source corresponds to a set of optical excitation filters individually movable in front of a given excitation radiation source so that radiation of the desired wavelength spectrum always falls on the measured surface, and an excitation filter controller for moving the optical excitation filters in front of the respective excitation radiation sources. In case of three broad-spectrum excitation radiation sources, these sources will be arranged evenly on a circle coaxial with the aperture of the excitation chamber at an angle of 120° and inclined at an angle of 45° to 60° to the surface; in practice, an angle of 53° has proved to be preferable. This, as mentioned above, minimizes unwanted reflectances, disturbing reflections from wet surfaces and other sources of optical artifacts, as well as ideally illuminating an uneven surface, thus minimizing shading by the measured surface's unevenness. In these alternative embodiments, the number and spectral transmittance of the optical excitation filters correspond to the number and spectral transmittance of the corresponding emission filters. The optical excitation filters can be arranged in a set, e.g. in a straight line, but will preferably be arranged in a circle, where the excitation filter controller will be a rotating cylinder.

The individual sets of optical excitation filters can be positioned manually and independently of one another and also independently of the set of optical emission filters. However, since it is necessary to ensure the correct selection of all optical excitation filters and their corresponding emission filter in each individual measurement, it is more suitable if the activation of the respective excitation radiation sources and the positioning of all filters in the sets are controlled by the connected control unit.

In an even more preferred embodiment, all optical excitation and emission filters are arranged on one common rotary filter controller that is formed by an outer ring with optical excitation filter positions, rotating under excitation radiation sources, and an inner ring with optical emission filter positions, coaxial with the outer ring and rotating in the field of view of the camera, which is located outside the axis of the rings. This ensures that the same combinations of optical excitation and emission filter positions will always be used in each measurement and after each rotation of the filter controller. Therefore, on the one hand, this solution minimizes the number of necessary excitation radiation sources while maintaining the selectivity of detection and minimizing unwanted optical artifacts and shading and, on the other hand, prevents the possibility of confusion of optical excitation and emission filters in individual measurements.

In case of a request for subsequent processing of data into an area image in the form of maps showing the arrangement and quantity of these individual groups of microorganisms on the screen at the measurement site, the device of the invention will further comprise an image data processing unit connected to the camera and its memory module and a screen (imaging device) connected to it for imaging the map. The device thus enables visualization directly in the field on the built-in screen.

In such an embodiment, the image processing algorithm is two-step. In the first step, it uses the HDR - High Dynamic Range - technique to combine an image sequence taken with different exposure times into a single image representing an area map of the emission response intensity of the examined sample. The conversion curves are determined during calibration using the Debevec algorithm. In the second step, a conversion GAM - Genealize Additive Model - function, which was optimised in the calibration step using robust regression techniques, is applied to each pixel of the emissivity map.

Preferably, the screen is designed as a touch screen so that it cannot only present information but also receive user instructions. In practice, the screen will preferably be housed in a screen frame that will be fixedly or movably connected to the main frame of the device. In this embodiment, the device is mobile and can be used for measuring, processing, and presenting data directly in the field without the need for sampling, damage to surfaces, and the need for laboratory processing of samples.

From a structural point of view, the camera and the servomotor of the device of the invention are put in the main frame, to the underside of which a cylinder with filters and excitation radiation sources is rotatably connected, and under this cylinder, in the lower part of the device, an excitation chamber cover is fixedly connected to the main frame, which further adjoins the housing of the device. In the upper part of the housing, a screen connected to the control unit is put for continuous imaging of the measurement results, and next to the screen, a support point with a pressure pad is arranged. The screen can be put in a subframe of the screen. The device of the invention further preferably comprises a pressure bearing that presses the rotary cylinder with filters so that it rotates in the desired position.

To ensure mobility, the device of the invention is embodied as compact, with a handle attached to the frame of the device. In such an embodiment, the device can be easily manipulated, transferred, and applied to the surface of the examined material.

The device can be powered from the public network or using accumulators. In case of a compact mobile embodiment, the electrical energy accumulator can be located in the handle of the device.

A basic characteristic of growth communities is their diversity, which greatly complicates their evaluation. It is common for the Aspergillus mould colony to be a target a few millimeters in diameter, meaning it is easy to miss it with instruments when using an apparatus with a smaller diameter probe, but it is an organism that destroys concrete, plastics, leather, fabrics, and paper. A significant advantage of the device of the invention is the ability to document the area distribution of organisms on surfaces and thus significantly improve the interpretation of measurements and document the natural heterogeneity. Another significant advantage of the solution of the invention is the large area of take compared to existing devices, thus affecting the natural heterogeneity of natural growth communities and thus further increasing the information value of the measured data achieved by designing an optical excitation chamber surrounded by a cover that defines an aperture for imaging the surface, preferably circular. When imaging a surface in a circle with a diameter of, for example, 13 cm, the risk that there is no detectable microorganism colony in this area is minimal. The device of the invention thus, compared to apparatuses that do not affect the area and its heterogeneity, reduces the number of necessary repeated measurements.

The device of the present invention is therefore able to distinguish and quantify important groups of organisms in terms of biocorrosion of materials instead of just detecting their presence. In addition, the camera's large field of view makes it possible to capture and image the area distribution and representation of colonies or clusters of detected organisms on the examined surface. The device is designed as a compact, portable, and sensitive device intended for easy use in situ without the need for mechanical intervention in the surface of the examined material.

### Description of the drawings

A summary of the invention is further clarified using exemplary embodiments thereof, which are described with reference to the accompanying drawings, in which:
Fig. 1 is a general schematic view of the device;
Fig. 2 is a side sectional view of the device showing the internal arrangement of the apparatus;
Fig. 3 is a detail of the movement of the emission filters above the excitation chamber enabling simultaneous detection and quantification of several groups of microorganisms in a single image;
Fig. 4 is a diagram of the sequence of image processing steps in the creation of a multispectral concentration map;
Fig. 5 is a schematic representation of the arrangement of the excitation filters and broad-spectrum radiation sources;
Fig. 6 is a schematic representation of a cylinder with a set of optical excitation filters moved in front of one broad-spectrum radiation source;
Fig. 7 is a section through the excitation chamber.

### Exemplary embodiments of the invention

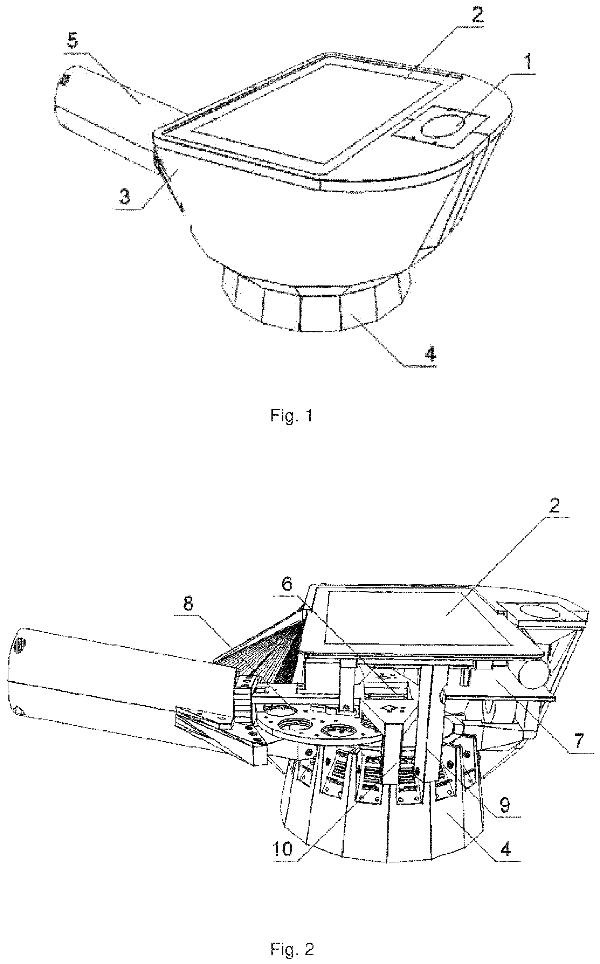

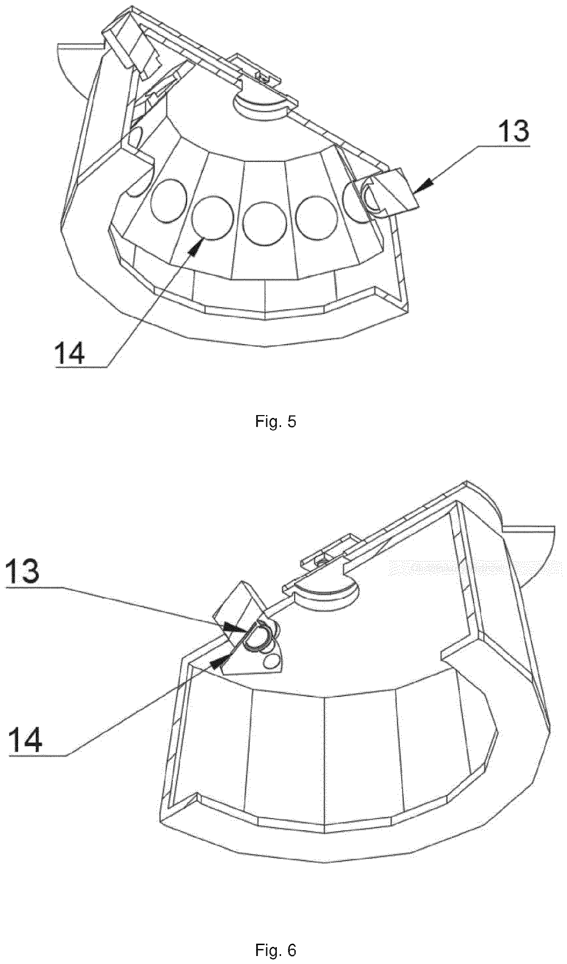

The invention will be further clarified using exemplary embodiments with reference to the respective drawings. The practical use, scheme of the apparatus and the method of data evaluation, high sensitivity and ability of differentiation of individual groups of microorganisms responsible for the biocorrosion of materials can be demonstrated by the exemplary embodiment shown in Figs. 1 to 4. Possible embodiments of the excitation radiation source and the set of optical excitation filters are schematically shown in Figs. 5 to 8.

Fig. 1 shows an overall schematic view of the device of the invention, from which it is clear that it is a compact mobile apparatus intended for the field. The device comprises a body 3, which is formed by a plastic cover, under which electronic and mechanical components are placed. The body 3 of the device extends into a handle 5, in which a battery/accumulator is stored. On the upper side of the body 3 of the apparatus, a touch screen 2 for controlling the device and possibly imaging continuous measurement results is placed. Next to the screen 2 opposite the handle 5, a pressure surface 1 is provided for the correct gripping and pressing of the device and ensuring correct contact of the cover of an excitation chamber 4 on the underside of the body 3 of the device with the measured surface.

Fig. 2 shows details of the internal arrangement of the device, in particular the optical excitation chamber 4 covered from the disturbing effects of ambient light, the handle 5 with an accumulator, and the touch screen 2 for setting parameters and monitoring the data measurement process. The touch screen 2 on the top of the device is mounted on a main frame 10 by means of a screen frame 9. Beneath the screen 2, the modules of a control unit 7 are stored. In the axis of the excitation chamber 4 passing through its top and perpendicular to the detected surface, a camera 6 and a cylinder 8 with emission filters are mounted at the top of the excitation chamber 4 in the main frame 10.

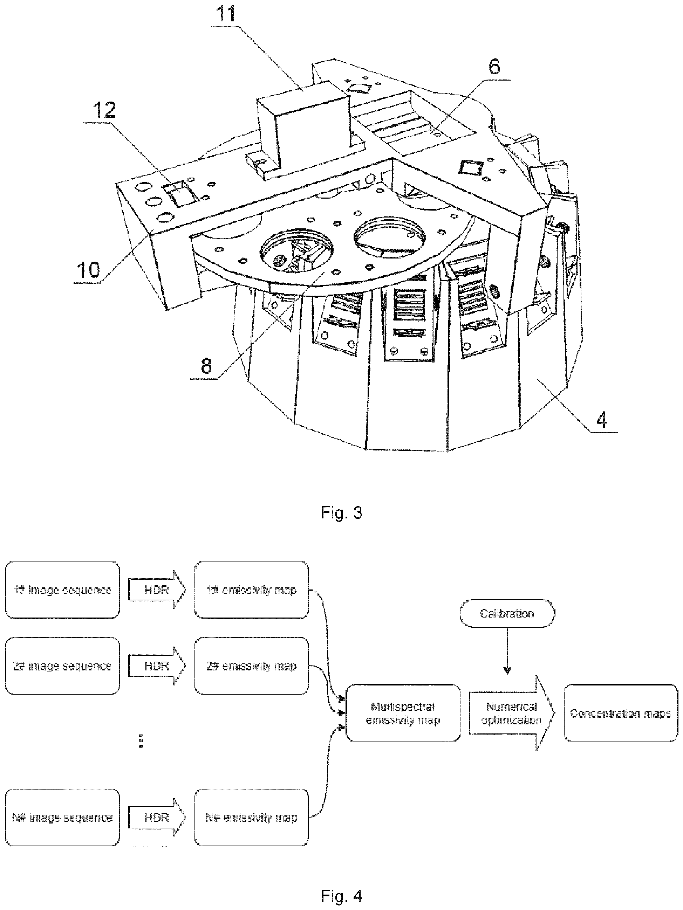

The actual detail of the device principle is shown in more detail in Fig. 3. The excitation chamber 4 is fixedly connected to the main frame 10. In the axis of the excitation chamber 4 passing through its top and perpendicular to the detected surface, the camera 6, which images the entire surface area of the material accessible through the excitation chamber 4 aperture in the lower part of the excitation chamber 4, where microorganisms are detected and quantified, is placed at the top of the excitation chamber 4. In the space between the lens of the camera 6 and the top of the excitation chamber 4, the circular cylinder 8 with optical emission filters rotates. The position of the cylinder with emission filters 8 is stabilized by means of a pressure bearing 12 put in the main frame 10.

The cover of the excitation chamber is divided into 16 fields around its circumference. 15 fields are provided with excitation radiation sources, the last field is provided with a common light source for taking standard photographs of the surface of the examined material. The excitation radiation sources are therefore arranged evenly on a circle coaxial with the aperture of the excitation chamber 4 and are inclined at an angle of 53° to the detected surface.

Arranged in the optical excitation chamber 4 are five different sources of excitation radiation suitable for the excitation of the detected microorganisms, wherein the same three excitation radiation sources for one type of microorganism are placed on a circle at an angle of approximately 120° to each other. The excitation radiation sources for exciting the detected surface of the material are, in the basic embodiment, LEDs emitting radiation of the desired wavelengths. Each type of excitation radiation source corresponds to exactly one optical emission filter in the circular cylinder 8. At least one position in the circular cylinder 8 is not occupied by an emission filter and thus transmits unfiltered light for taking standard photographs in case of activation of a conventional light source.

In a specific embodiment, the algae detection device uses autofluorescence of photosynthetic pigments at a combination of an excitation wavelength of 465 nm and an emission filter transmitting a wavelength of 680 nm, in case of cyanobacteria, an excitation wavelength of 595 nm and an emission filter for a wavelength of 650 nm are suitable, in case of diatoms, an excitation wavelength of 520 nm, an emission filter for a wavelength 680 nm are suitable. For the detection of moulds, the examined surface is stained using the fluorogenic reagent calcofluor and an excitation wavelength of 365 nm and an emission filter for a wavelength of 420 nm are used. For the detection of bacteria, the examined surface is stained with the fluorogenic reagent danzylhydrazine excited with radiation of a wavelength of 365 nm and an emission filter transmitting a wavelength of 520 nm is used. In addition, mosses are distinguishable in a macroscopic photography by image analysis and spectra identical to those for algae.

The setting of the required optical emission filter to the correct position in front of the lens of the camera 6 is ensured by a servomotor 11. The control unit 7 takes care of the coordination of the setting of the required optical emission filter, activation of the corresponding excitation radiation source, and imaging of images by the camera 6.

Figs. 4 then shows the process of processing and integrating the sequences of the acquired images while creating a multispectral concentration map over the entire measured area. After the measurement is started, an image sequence is automatically recorded by the camera 6 for each measured combination of excitation light and fluorescence emission at different brightness and contrast settings. Through the HDR (High Dynamic Range) technology, an emissivity map is then created from the images. The partial emissivity maps are then put together to form a multispectral emissivity map, which is optimized in the next step and, according to the inputted calibration, converted to a map of the distribution of cell/tissue concentrations of the organism/organisms of interest on the examined surface area.

Fig. 5 schematically shows an optical excitation chamber 4 of the device in an embodiment in which a set of three universal broad-spectrum excitation radiation sources 13 is evenly arranged around the circumference of the excitation chamber 4, wherein each universal broad-spectrum excitation radiation source 13 corresponds to a set of optical excitation filters individually movable in front of a given source of excitation radiation so that radiation with the required spectrum of wavelengths always falls on the measured surface. An excitation filter controller is further embodied in the excitation chamber for moving the optical excitation filters in front of the respective excitation radiation sources.

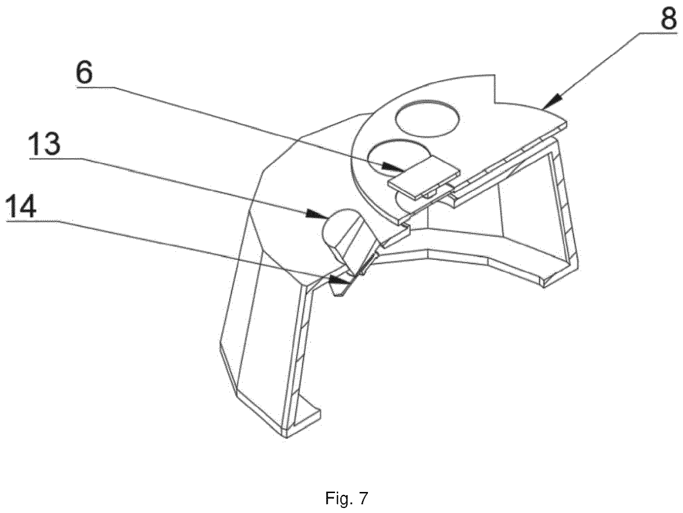

Another possible embodiment of the device of the invention is then schematically shown in Fig. 6 for illustration only. The excitation chamber is provided with a cylinder 14 with a set of optical excitation filters moved in front of one broad-spectrum excitation radiation source 13. Fig. 7 then shows a section through this excitation chamber 4. To image uneven surfaces, more of these broad-spectrum excitation radiation sources 13 with cylinders 14 moved in front with sets of optical excitation filters can be arranged in the excitation chamber 4.

An alternative, not shown, may then be an embodiment where all optical excitation and emission filters are arranged on one common rotary filter controller that is formed by an outer ring with optical excitation filter positions, rotating under excitation radiation sources, and an inner ring with optical emission filter positions, coaxial with the outer ring and rotating in the field of view of the camera, which is located outside the axis of the rings.

The apparatus can be used on any type of surface - mortar, concrete, stone, uneven paving, glossy tiles, wood, iron structures, free soil surface, polycarbonates, etc. The minimum area suitable for the proper application of the cover (of the excitation chamber) is a circle with a diameter of 13 cm, or a square with a side length of 13 cm, wherein the camera's field of view is a circle approximately 10 cm in diameter. The camera 6 of the apparatus is ideally focused on the plane of the application surface of the cover. However, the depth of field of the camera 6 allows the apparatus to be used with sufficient accuracy also on surfaces raised or sunken in the range of approximately 2.5 cm against the plane of the application surface of the cover, in the axis of the detection camera 6, which images the entire area where microorganisms are detected and quantified on the surface of materials, the servomotor sets a sequence of emission filters enabling optical differentiation of individual groups of organisms, the process of processing and integration of sequences of acquired images when creating a multispectral concentration map over the entire measured area. Therefore, the camera 6 is set at such a setting and distance from the detected material surface as to image at least 70 cm² of the detected material surface area.

The edge of the cover is provided with a collar made of a foam material, which is able to adapt to the shape of a slightly granular or slightly uneven surface. During measurements on such a surface, the device must be pressed against the measured surface by means of a handle 5 and a marked support point, for example, a pressure pad 1 on the opposite side of the device body 3, so that no light from outside enters the cover.

The measured surface should be moist so that all biofilm-forming organisms are physiologically active. During the measurement, a place that represents the given locality, the given situation, is carefully selected and the surface measurement is performed - first directly, eventually after additional moistening of the surface, and after spraying fluorescent dye solutions using calcofluor and danzyl-hydrazine.

### Industrial applicability

The device according to the invention is intended for early warning, control, and preventive detection and quantification of organisms causing biocorrosion of materials. Specifically, the device can be used for preventive inspections of concrete structures of bridges, dams, cooling towers, and other structures as well as for the evaluation of microbial settlement of facade surfaces, statues, stone, wood, plastic insulation of switchboards and in all cases where we need to detect the presence of microorganisms, which can disintegrate, decompose, and degrade the material and thus significantly reduce its life, in time. Alternatively, the device can be used, for example, in water management, where, for example, in the water industry, it is necessary to detect whether moulds or cyanobacteria are present during the production of drinking water, e.g. on water reservoir walls, filter walls, and distribution network elements, or also in research where e.g. toxic substances are decomposed by microbial biofilms and the composition of microorganism communities needs to be evaluated in a non-destructive manner.

### List of reference signs

1. Pressure pad
2. Screen
3. Body
4. Excitation chamber
5. Handle with an accumulator
6. Camera
7. Control unit
8. Cylinder with emission filters
9. Screen frame
10. Main frame
11. Servomotor
12. Pressure bearing
13. Broad-spectrum excitation radiation source
14. Cylinder with excitation filters

### Literature:

Boniek, D., Mendes, I.D., Paiva, C.A.O., Lana, U.G.D., dos Santos, A.F.B., Stoianoff, M.A.D., 2017. Ecology and identification of environmental fungi and metabolic processes involved in the biodeterioration of Brazilian soapstone historical monuments. Letters in Applied Microbiology 65, 431-438.
Gaylarde, C., Baptista-Neto, J.A., Tabasco-Novelo, C., Ortega-Morales, O., 2018. Weathering of granitic gneiss: A geochemical and microbiological study in the polluted sub-tropical city of Rio de Janeiro. Science of the Total Environment 644, 1641-1647.
Gaylarde, C., Ogawa, A., Beech, I., Kowalski, M., Baptista-Neto, J.A., 2017. Analysis of of dark crusts on the church of Nossa Senhora do Carmo in Rio de Janeiro, Brazil, using chemical, microscope and metabarcoding microbial identification techniques. International Biodeterioration & Biodegradation 117, 60-67.
C. Gaylarde, M. Ribas Silva, Th. Warscheid: Microbial impact on building materials: an overview, Materials and Structures, 2003, Volume 36, Number 5, Page 342
Gonzalez-Perez, M., Brinco, C., Vieira, R., Rosado, T., Mauran, G., Pereira, A., Candeias, A., Caldeira, A.T., 2017. Dual phylogenetic staining protocol for simultaneous analysis of yeast and bacteria in artworks. Applied Physics a-Materials Science & Processing 123, 11.
M. Gonzalez, R. Vieira, P. Nunes, T. Rosado, S. Martins, A. Candeias, A. Pereira and A. T. Caldeira, Fluorescence In Situ Hybridization: a potentially useful technique for detection of microorganisms on mortars, e-conservation Journal 2, 2014, pp. 44-52
Mejia, E., Tobon, J.I., Osorio, W., 2019. Urban structure degradation caused by growth of plants and microbial activity. Materiales De Construccion 69.
Nuhoglu Y, Var M, Koçak E, Uslu H, Demir H (2017) In Situ Investigation of the Biodeteriorative Microorganisms Lived on Stone Surfaces of the Sumela Monastery (Trabzon, Turkey). J Environ Anal Toxicol 7: 506.
Sanmartin, P., DeAraujo, A., Vasanthakumar, A., 2018. Melding the Old with the New: Trends in Methods Used to Identify, Monitor, and Control Microorganisms on Cultural Heritage Materials. Microbial Ecology 76, 64-80.
Sohrabi, M., Favero-Longo, S.E., Perez-Ortega, S., Ascaso, C., Haghighat, Z., Talebian, M.H., Fadaei, H., de los Rios, A., 2017. Lichen colonization and associated deterioration processes in Pasargadae, UNESCO world heritage site, Iran. International Biodeterioration & Biodegradation 117, 171-182.
Warscheid, T., Braams, J., 2000. Biodeterioration of stone: a review. International Biodeterioration & Biodegradation 46, 343-368.
Zanardini, E., May, E., Purdy, K.J., Murrell, J.C., 2019. Nutrient cycling potential within microbial communities on culturally important stoneworks. Environmental Microbiology Reports 11, 147-154.

## Claims

1. A mobile device for non-destructive fluorescence differentiation, imaging, and quantification of microorganisms on the surface of materials, comprising
- an excitation radiation source for exciting the detected surface of the material and
- a camera (6) for fluorescent imaging of the excited surface of the material provided with a memory module for storing images from the camera (6),
**characterized in that** it further contains an excitation chamber (4) with an aperture for placement on the detected material surface, in which a set of excitation radiation sources, a camera (6), a set of optical emission filters movable individually into the field of view of the camera (6) and transmitting wavelengths corresponding to the fluorescence signals elicited by the excitation radiation of the excitation radiation sources and characteristic of individual groups of organisms, and an emission filter controller for moving the optical emission filters into the field of view of the camera (6), are enclosed.

2. The mobile device for non-destructive fluorescence differentiation, imaging, and quantification of microorganisms on the surface of materials according to claim 1, **characterized in that** the optical emission filters are arranged in a set in a circle, wherein the emission filter controller is a rotary cylinder (8).

3. The mobile device for non-destructive fluorescence differentiation, imaging, and quantification of microorganisms on the surface of materials according to claim 1 or 2, **characterized in that** the excitation chamber (4) is surrounded by a cover made of a flexible dark material without shape memory.

4. The mobile device for non-destructive fluorescence differentiation, imaging, and quantification of microorganisms on the surface of materials according to any one of the preceding claims, **characterized in that** the excitation radiation sources are arranged evenly above the excitation chamber (4) aperture in a circle coaxial with the excitation chamber (4) aperture, and they are inclined at an angle of 45° to 60° towards the surface.

5. The mobile device for non-destructive fluorescence differentiation, imaging, and quantification of microorganisms on the surface of materials according to any one of the preceding claims, **characterized in that** the set of excitation radiation sources is a set of universal broad-spectrum excitation radiation sources (13), wherein each universal broad-spectrum excitation radiation source (13) corresponds to a set of optical excitation filters individually movable in front of a given excitation radiation source, wherein the mobile device further comprises an excitation filter controller for moving the optical excitation filters in front of the respective excitation radiation sources and a control unit (7) for activating the respective excitation radiation sources and replacing the emission filters.

6. The mobile device for non-destructive fluorescence differentiation, imaging, and quantification of microorganisms on the surface of materials according to claim 5, **characterized in that** the optical excitation and emission filters are arranged on one common rotary filter controller that is formed by an outer ring with optical excitation filter positions, rotating under the excitation radiation sources, and an inner ring with optical emission filter positions, coaxial with the outer ring and rotating in the field of view of the camera (6), which is located outside the axis of the rings.

7. The mobile device for non-destructive fluorescence differentiation, imaging, and quantification of microorganisms on the surface of materials according to any one of the preceding claims, **characterized in that** it is provided with a screen (2) connected to a control unit (7) for continuously imaging the measurement results.

8. The mobile device for non-destructive fluorescence differentiation, imaging, and quantification of microorganisms on the surface of materials according any one of the preceding claims, **characterized in that** it is provided with a handle (5).
